Europäisches Patentamt

European Patent Office

Office européen des brevets

(19)

(11) Veröffentlichungsnummer: **0 035 649**
**B1**

(12) **EUROPÄISCHE PATENTSCHRIFT**

(45) Veröffentlichungstag der Patentschrift:
**20.07.83**

(21) Anmeldenummer: **81100825.9**

(22) Anmeldetag: **05.02.81**

(51) Int. Cl.³: **C 07 C 45/86,** C 07 C 49/92,
B 01 J 31/22

(54) **Flüssige, kältestabile Titan-Katalysatorzubereitung und Verfahren zu deren Herstellung.**

(30) Priorität: **04.03.80 DE 3008193**

(43) Veröffentlichungstag der Anmeldung:
**16.09.81 Patentblatt 81/37**

(45) Bekanntmachung des Hinweises auf die Patenterteilung:
**20.07.83 Patentblatt 83/29**

(84) Benannte Vertragsstaaten:
**AT BE CH DE FR GB IT LI LU NL SE**

(56) Entgegenhaltungen:
**US-A-3 682 688**
**US-A-3 888 895**
**CHEMICAL ABSTRACTS, Band 79, Nr. 19, 12. November**
**1973, Seite 64, Nr. 116690k Columbus, Ohio, U.S.A.**

(73) Patentinhaber: **DYNAMIT NOBEL**
**AKTIENGESELLSCHAFT, Patentabteilung**
**Postfach 1209, D-5210 Troisdorf, Bez. Köln (DE)**

(72) Erfinder: **Barfurth, Dieter, Weidchensweg 24,**
**D-5204 Lohmar (DE)**
Erfinder: **Nestler, Heinz, Dr., Rembrandtstrasse 73,**
**D-5210 Troisdorf-Eschmar (DE)**

## Flüssige, kältestabile Titan-Katalysatorzubereitung und Verfahren zu deren Herstellung

Gegenstand der vorliegenden Erfindung ist eine Lösung von Diisopropoxy-bis(2,4-pentandionato)-titan(IV)-Lösung in Isopropanol, die auch bei Temperaturen bis zu –20 °C stabil ist.

Diisopropoxy-bis(2,4-pentandionato)-titan (IV) ist eine an sich bekannte Verbindung, die z.B. als Vernetzer, Haftvermittler oder Katalysator eingesetzt wird. Von den Tetraalkoxytitanaten unterscheidet sie sich insbesondere durch eine – häufig erwünschte – geringere Reaktivität und durch die Tatsache, dass ihre Hydrolyse stufenweise verläuft und – unter Bildung einer Titanylverbindung – zunächst nur die Alkoxygruppen als Isopropanol freigesetzt werden (vgl. A. Yamamoti, S. Kambara, J.Am.Chem.Soc. 79 (1957) 4344-4348). Um Diisopropoxy-bis(2,4-pentandionato)-titan (IV), das in reiner Form einen orangen Feststoff darstellt (vgl. D.M. Puri et al, J. Less-Common Metals, 4 (1962), 393–398), als Katalysator oder Vernetzer einfacher handhaben zu können, wird in der Regel seine isopropanolische Lösung so eingesetzt, wie sie bei der üblichen Herstellung durch Reaktion von Tetraisopropyltitanat mit Acetylaceton im Molverhältnis 1:2 anfällt. Unter der Bezeichnung Titanacetylacetonat wird gewöhnlich diese Lösung verstanden, die sich im allgemeinen aus 75,2 Gew.-% Titanverbindung und 24,8 Gew.-% Isopropanol zusammensetzt. Bei den bekanntgewordenen Anwendungen dieser Katalysatorlösung stört der Alkohol nicht, oder er wird vor der Anwendung der Titanverbindung unter Austausch gegen ein anderes Lösungsmittel destillativ entfernt.

Während der kalten Jahreszeit treten bei der Lagerung, der Förderung durch Pumpen und Rohrleitungen und der Dosierung dieser Katalysatorzubereitung folgende Nachteile auf: In den Gebinden, Rohren und Dosiereinrichtungen setzen sich hin und wieder feste Ausscheidungen ab. Diese Niederschläge lassen sich zwar durch Erwärmen wieder beseitigen, jedoch beansprucht dieser Prozess einen unnötigen Zeitaufwand und verursacht zusätzliche Manipulationen und Kosten. Nach dem Aufschmelzen können sich dabei, besonders in Gebinden, unerwünschte Konzentrationsunterschiede ausgebildet haben.

Zur Ausschaltung dieser störenden Erscheinungen ist es bereits versucht worden, durch Zusatz weiteren Isopropanols, anderer Alkohole oder auch anderer Lösungsmittel Abhilfe zu schaffen. Es stellte sich aber bei der Lagerung von Versuchsansätzen bei einer Prüftemperatur von –18 °C heraus, dass viele Verdünnungsmittel auch bei relativ grossen Zusätzen wirkungslos blieben: Eine Niederschlagsbildung bei tiefen Temperaturen konnte nicht verhindert werden. Bei manchen Lösungsmitteln, wie z.B. Methanol oder 35 Äthanol, ergaben Zusatzmengen von mindestens 10% bezogen auf die Ausgangsmischung, zwar Standzeiten von länger als 20 Wochen bei –20 °C. Solche hohen Dosierungen bedeuten aber, dass der Alkoholgehalt der Ausgangsmischung von 24,8% auf dann 31,6% ansteigt und der Titangehalt, ausgedrückt als $TiO_2$, von ursprünglich 16,5% $TiO_2$

auf ca. 15% $TiO_2$ absinkt. Dies bedeutet jedoch eine ziemlich starke, ungewünschte Veränderung des Produktes.

Es bestand deshalb die Aufgabe, Lösungen von Diisopropoxy-bis(2,4-pentandionato)-titan(IV) in Isopropanol derart zu stabilisieren, dass auch bei Temperaturen bis herab zu –20 °C kein Niederschlag ausfällt und der Titangehalt der Lösung durch das Stabilisierungsmittel nicht wesentlich herabgesetzt wird.

In Erfüllung dieser Aufgabe wurde nun gefunden, dass der Zusatz einer genau definierten, geringen Menge Wasser, nämlich 0,05 bis 0,15 Mol Wasser pro Mol Titanatom, Titanacetylacetonatlösungen kältestabilisiert. Auf diese Weise behandelte Titanacetylacetonatlösungen erwiesen sich bei Langzeitlagerungen bei –18 °C als absolut stabil und blieben klar.

Gegenstand der vorliegenden Erfindung sind also Lösungen von Diisopropoxy-bis (2,4-pentandionato)-titan(IV) in Isopropanol, die durch einen Zusatz von 0,05 bis 0,15 Mol Wasser pro Titanatom gekennzeichnet sind.

Die erfindungsgemässe Lösung ist in ihren Eigenschaften und ihrer Wirksamkeit praktisch unverändert gegenüber der wasserfreien Lösung. Bei einem Wasserzusatz von 0,1 Mol $H_2O$ /Titanatom erhöht sich der Alkoholgehalt der Mischung zwar um 2,5%, der Gehalt an Ti, ausgedrückt als $TiO_2$, vermindert sich jedoch nur von 16,53 auf 16,47%.

Wasserzusätze, die unter der angegebenen unteren Grenze liegen, zeigen einen deutlich verminderten Effekt; grössere Zusatzmengen als angegeben führen auch schon bei Raumtemperatur zu baldigen Niederschlägen.

Die stabilisierende Wirkung des erfindungsgemässen Wasserzusatzes zeigt sich nicht nur bei 75%igen Titanacetylacetonatlösungen in Isopropanol, sondern auch bei Lösungen, bei denen der Gehalt der Titanverbindung zwischen 65 und 80% schwankt, d.h. bei Katalysatorlösungen mit einem aktiven $TiO_2$-Gehalt zwischen etwa 14,3 und 17,6%.

Die Herstellung der erfindungsgemässen, kältestabilen Katalysatorlösungen kann auf verschiedenen einfachen, völlig unproblematischen Wegen geschehen. Dabei setzt man entweder die benötigte Menge Wasser bei der Zubereitung der Titanacetylacetonatlösung mit hinzu, wobei zu beachten ist, dass nicht die gesamte Menge Wasser unmittelbar mit dem vorgelegten Isopropyltitanat reagieren kann. Zweckmässigerweise löst man das Wasser also im Acetylaceton vor dessen Zudosierung. Der andere Weg besteht darin, der fertigen Titanacetylacetonatlösung die notwendige Menge Wasser hinzuzufügen und die Mischung gegebenenfalls eine zeitlang zu erwärmen.

Die Herstellungsverfahren und die Ausprüfung der Produkte samt Ergebnissen werden in den folgenden Beispielen näher erläutert.

**Beispiel 1**
Herstellung der Katalysatorlösung unter Zusatz von Wasser

In einem 1 l-Dreihalskolben, der mit Thermometer, Rührer und Tropftrichter versehen ist, werden 284 g (= 1 Mol) Isopropyltitanat vorgelegt; über den Tropftrichter wird eine Mischung aus 200 g (= 2 Mol) Acetylaceton und 1,8 g (= 0,1 Mol) Wasser so zudosiert, dass in der Reaktionsmischung eine Temperatur von 60 °C nicht überschritten wird. Nach beendeter Zugabe des Acetylaceton-Wasser-Gemischs wird eine orangerote Flüssigkeit erhalten, die nach Austausch des Tropftrichters gegen einen Rückflusskühler zur Vervollständigung der Reaktion 1 Stunde zum Rückfluss erhitzt wird.

**Beispiel 2**
Modifizierung einer Katalysatorlösung durch Zusatz von Wasser

In einem 1 l-Rundkolben werden 484 g einer handelsüblichen, 75%igen Titanacetylacetonatlösung in Isopropanol eingewogen. Diese Menge enthält 1 Mol reines Diisopropoxytitan-bis-acetylacetonat. Dazu werden 1,8 g (= 0,1 Mol) Wasser gegeben, das sich sofort darin löst. Diese Mischung wird anschliessend 1 Stunde zum Rückfluss erhitzt.

**Beispiel 3**
Prüfung des Kristallisationsverhaltens bei niederen Temperaturen

Zur Untersuchung der Kristallisationsneigung der Katalysatorlösungen bei Temperaturen vorzugsweise unterhalb des Gefrierpunktes von Wasser wird eine Probe des zu untersuchenden Materials in einem Tiefgefrierfach eines Kühlschrankes gelagert. Die Temperatur schwankt an diesem Lagerort zwischen −16 und −20 °C und liegt im Mittel bei −18 °C.

In der nachfolgenden Tabelle sind die Ergebnisse der Kältelagerungen der Katalysatorlösungen mit verschiedenen Wasserzusätzen angegeben. (Herstellungsverfahren jeweils gemäss Beispiel 1 bzw. 2).

| Wasserzusatz in Mol $H_2O$ pro Ti-Atom | Kristallisationsverhalten bei −18 °C | |
|---|---|---|
| ohne | Herstellung n. Bsp. 1 | Herstellung n. Bsp. 2 |
| | nach 24 Stunden kristallisiert | |
| 0,025 Mol/Ti | nach 3 Tagen krist. | nach 4 Tagen krist. |
| 0,05 Mol/Ti | nach 3 Monaten noch in Ordnung | nach 3 Monaten noch in Ordnung |
| 0,10 Mol/Ti | nach 3 Monaten noch in Ordnung | nach 3 Monaten noch in Ordnung |
| 0,15 Mol/Ti | nach 3 Monaten noch in Ordnung | nach 3 Monaten noch in Ordnung |
| 0,20 Mol/Ti | bildet nach 10 Tagen bei Zimmertemperatur einen Niederschlag | bildet nach 14 Tagen bei Zimmertemperatur einen Niederschlag |

**Patentansprüche**

1. Kältestabile, katalytisch wirksame, Lösung von Diisopropoxy-bis(2,4-pentandionato)-titan-(IV) in Isopropanol dadurch gekennzeichnet, dass der Lösung Wasser in Mengen von 0,05 bis 0,15 Mol, bezogen auf 1 Mol Titanverbindung, zugeführt ist.

2. Verfahren zur Herstellung einer Katalysatorlösung gemäss Anspruch 1, dadurch gekennzeichnet, dass die Wassermenge bereits bei der an sich bekannten Herstellung der Diisopropoxy-bis(2,4-pentandionato)-titan(IV)-Lösung aus Tetraisopropyltitanat und Acetylaceton mit eingebracht wird.

3. Verfahren zur Herstellung einer Katalysatorlösung gemäss Anspruch 1, dadurch gekennzeichnet, dass die Wassermenge der in einem vorher durchgeführten Arbeitsgang hergestellten Diisopropoxy-bis(2,4-pentandionato)-titan(IV)-Lösung zugefügt wird.

**Revendications**

1. Solution de diisopropoxy-bis (2,4-pentanedionato)-titane (IV) dans de l'isopropanol, catalytiquement active et stable au froid, caractérisée en ce que la solution est additionnée d'eau en des quantités de 0,05 à 0,15 mole par rapport á 1 mole de composé de titane.

2. Procédé pour la préparation d'une solution de catalyseur selon la revendication 1, caractérisé en ce que la quantité d'eau est déjà introduite au cours de la préparation conue en soi de la solution de diisopropoxy-bis(2,4-pentanedionato)-titane (IV) á partir de titanate de tétraisopropyle et d'acétylacétone.

3. Procédé pour la préparation d'une solution de catalyseur selon la revendication 1, caractérisé en ce que la quantité d'eau est ajoutée à la solution de diisopropoxy-bis (2,4-pentanedionato)-titane (IV) préparée au cours d'une phase opératoire réalisée au préalable.

## Claims

1. Cold stable, catalytically active, solution of diisopropoxy-bis(2,4-pentandedionato)-titanium-(IV) in isopropanol, characterised in that the solution is added to water in amounts of 0.05 to 0.15 mole, related to 1 mole of titanium compound.

2. Process for the production of a catalyst solution according to claim 1, characterised in that the amount of water is already employed in the production, known per se, of diisopropoxy-bis-(2,4-pentanedionato)-titanium(IV) solution from tetraisopropyl titanate and acetylacetone.

3. Process for the production of a catalyst solution according to claim 1, characterised in that the amount of water is added to the diisopropoxy-bis(2,4-pentanedionato)-titanium(IV) solution produced in a previously carried out working operation.